Europäisches Patentamt

European Patent Office

Office européen des brevets

(11) Veröffentlichungsnummer: **0 287 893 B1**

(12) **EUROPÄISCHE PATENTSCHRIFT**

(45) Veröffentlichungstag der Patentschrift: **28.10.92**

(21) Anmeldenummer: **88105477.9**

(22) Anmeldetag: **06.04.88**

(51) Int. Cl.⁵: **H01R 13/66**

(54) **Pneumatischer und elektrischer Steckanschluss für Saugelektroden.**

(30) Priorität: **21.04.87 DE 8705787 U**

(43) Veröffentlichungstag der Anmeldung:
**26.10.88 Patentblatt 88/43**

(45) Bekanntmachung des Hinweises auf die
Patenterteilung:
**28.10.92 Patentblatt 92/44**

(84) Benannte Vertragsstaaten:
**AT BE CH DE GB LI NL**

(56) Entgegenhaltungen:
**DE-B- 1 296 231**
**US-A- 3 195 095**

(73) Patentinhaber: **SIEMENS AKTIENGESELL-
SCHAFT
Wittelsbacherplatz 2
W-8000 München 2(DE)**

(72) Erfinder: **Nützel, Karl-Heinz
In der Reuth 102
W-8520 Erlangen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung bezieht sich auf einen pneumatischen und elektrischen Steckanschluß zum Anschließen von Saugelektroden an ein Bedienungsgerät, insbesondere für Reizstromtherapie.

Saugelektroden, insbesondere solche in der Reizstromtherapie, umfassen bekanntlich für den pneumatischen Teil und den elektrischen Teil der Saugelektrode unterschiedliche Steckanschlüsse am Bedienungsgerät.

Aus der DE-B-12 96 231 ist eine Steckverbindung zur Verbindung von Saugelektroden mit einer Vakuumleitung bekannt, die an eine Vakuumeinrichtung eines elektromedizinischen Gerätes angeschlossen ist. Die bekannte Steckverbindung besteht einerseits aus einer aus elektrisch leitendem Material bestehenden und mit einem Ventil versehenen Konushülse, die endseitig an einen Schlauch angesetzt ist, der die Vakuumleitung zum elektromedizinischen Gerät bildet. Im Inneren des Schlauches ist eine elektrische Leitung geführt, die leitend mit der Konushülse verbunden ist. Andererseits besteht die Steckverbindung aus einem mit der Konushülse koppelbaren Konusstecker - aus ebenfalls elektrisch leitfähigem Material -, der an der Saugelektrode befestigt ist. Beim Kuppeln des Steckers mit der Konushülse wird das Ventil der Konushülse geöffnet und die Saugelektrode ist über eine Bohrung des Steckers an die Vakuumleitung anschließbar. Gleichzeitig wird die Saugelektrode über den Stecker und die Konushülse an die Stromzuleitung angeschlossen. Die bekannte Einfachsteckverbindung ist an der Saugelektrode anzubringen und befindet sich somit unmittelbar im Handhabungsbereich der Saugelektrode durch die Bedienungsperson. Dies kann sowohl zum ständigen unbeabsichtigten Lösen der Kupplung als auch zu Schwierigkeiten bei der elektrischen Abschirmung der Behandlungsperson und des Patienten führen.

Zur Verbindung zweier Schläuche, die der Sauerstoffversorgung von fliegendem Personal dienen, ist aus der US-A-3 195 095 eine Kupplung, bestehend aus einem männlichen Steckerteil und einem weiblichen Gegensteckerteil, bekannt. Die Steckerteile werden dabei an den miteinander zu verbindenden Enden zweier Schläuche befestigt, wobei ein Rohrteil des Steckerteils in eine Buchse des Gegensteckerteils unter Verrastung einsetzbar ist und dadurch ein Ventil des Gegensteckerteils für den Sauerstoffdurchlaß geöffnet wird. In oder an den Schläuchen ist ferner eine elektrische Leitung verlegt. Zur Kontaktierung dieser Leitungen weist die Kupplung ebenfalls männliche und weibliche elektrische Steckerteile auf.

Aufgabe vorliegender Erfindung ist es, einen Steckanschluß der eingangs genannten Art zu vereinfachen.

Erfindungsgemäß wird dies bei einem Steckanschluß mit den Merkmalen des Patentanspruches 1 erreicht. Der Steckanschluß gemäß der Erfindung vereinigt den pneumatischen und elektrischen Teil in einem einzelnen elektrischen Stecker, der mit einem zugehörigen Gegensteckteil des Bedienungsgerätes kuppelbar ist.

Weitere Vorteile und Einzelheiten der Erfindung ergeben sich aus der nachfolgenden Beschreibung eines Ausführungsbeispiels anhand der Zeichnung und in Verbindung mit den Ansprüchen 2-4.

Es zeigen:

Figur 1 einen Steckanschluß gemäß der Erfindung,

Figur 2 einen pneumatischen und elektrischen Schlauchanschluß für ein einpoliges Kabel,

Figur 3 einen Querschnitt III-III durch das Kabel der Figur 2,

Figur 4 einen pneumatischen und elektrischen Schlauchanschluß für ein mehrpoliges elektrisches Kabel und

Figur 5 einen Querschnitt V-V durch das Kabel der Figur 4.

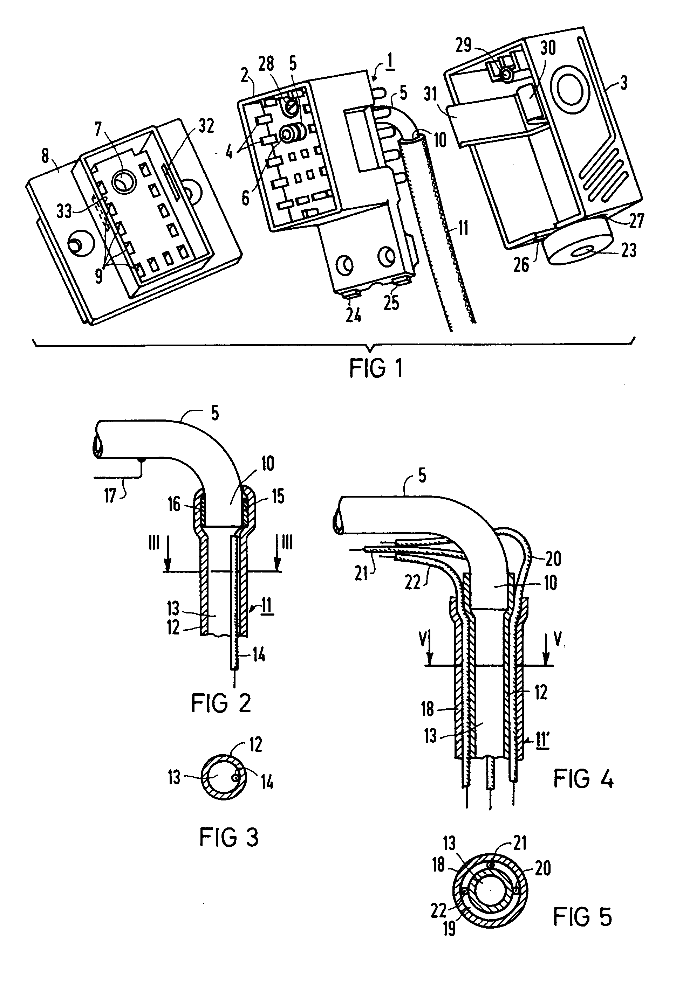

In der Figur 1 umfaßt ein elektrischer Mehrfachstecker 1 ein proximales Gehäuseteil 2, auf das ein distales Rückenteil 3 ansteckbar ist. Das proximale Gehäuseteil 2 umfaßt Steckstifte 4 (elektrische Steckkontakte) und einen pneumatischen Rohranschluß 5. Das Ende 6 des pneumatischen Rohranschlusses 5 paßt dichtend in einen pneumatischen Gegenanschluß 7 in einem Gegensteckteil 8 für den elektrischen Mehrfachstecker 1. Das Gegensteckteil 8 ist am (nicht dargestellten) Bedienungsgerät, insbesondere Reizstromtherapiegerät, montiert. Die Buchsen 9 sind weibliche Gegensteckbuchsen für die Steckstifte 4 des Steckers 1.

Am Ende 10 des pneumatischen Rohranschlusses 5 ist ein Kombinationsschlauch 11 oder 11' angeschlossen, der entsprechend dem Schlauch in der Figur 2 oder jenem in der Figur 4 ausgebildet sein kann.

Dementsprechend kann also gemäß der Figur 2 der Kombinationsschlauch 11 ein pneumatisches Schlauchteil 12 umfassen, das einerseits mit dem pneumatischen Teil der (nicht dargestellten) Saugelektrode und andererseits mit dem pneumatischen Rohranschluß 5 pneumatisch in Verbindung steht. Im pneumatischen Schlauchteil 12 (im vorliegenden Teil im hohlen Inneren 13 des Schlauchteiles 12) ist eine einpolige elektrische Leitung 14 für den elektrischen Teil der (nicht dargestellten) Saugelektrode eingelagert. Die am pneumatischen Rohranschluß 5 liegende Metallader 15 der einpoligen elektrischen Leitung 14 kontaktiert direkt elektrisch mit dem metallischen pneumatischen Rohranschluß 5. Als kontaktunterstützendes Andruckmittel dient eine Andruckmuffe 16, über die dann das Schlauchteil 12 geschoben ist. Zur Weitergabe der elektrischen Verbindung kann dann der metallische pneumatische Rohranschluß 5 einen elektrischen Anschluß 17 direkt am proximalen Gehäjseteil 2 dem elektrischen Mehrfachsteckers 1 haben.

Im Falle der Figuren 4 und 5 handelt es sich um einen dreipoligen (oder noch mehrpoligeren) Anschluß. Hier ist über das pneumatische Schlauchteil 12 mit dem hohlen Innenraum 13 ein zweites (elektrisches) Schlauchteil 18 gezogen, das einen Zwischenraum 19 freiläßt, in dem z.B. drei elektrische Leitungen 20, 21 und 22 für einen z.B. dreipoligen Anschluß verlaufen.

Zur Montage des distalen Rückenteils 3 auf dem proximalen Gehäuseteil 2 wird der Mehrfachschlauch 11 bzw. 11' vor Anschluß an dem pneumatischen Rohranschluß 5 durch die Öffnung 23 (Staubschutzkappe) des distalen Rückenteils 3 geschoben. Nach dem Anschluß des Kombinationsschlauches 11 bzw. 11' am pneumatischen Rohranschluß 5 wird das distale Rückenteil 3 am proximalen Gehäuseteil 2 so angesetzt, daß Rastnasen 24, 25 am proximalen Gehäuseteil 2 in zugehörige Rastschlitze 26, 27 am proximalen Gehäuseteil 3 einrasten. Durch Festziehen einer Schraube 28 am proximalen Gehäuseteil 2 des elektrischen Mehrfachsteckers 1, die in ein Gewindeloch 29 am distalen Rückenteil 3 paßt, ist jetzt das distale Rückenteil 3 fest mit dem proximalen Gehäuseteil 2 verbunden. Der elektrische Mehrfachstecker 1 mit dem pneumatischen Rohranschluß 5 ist jetzt betriebsbereit und kann in das Gegensteckteil 8 am (nicht dargestellten) Betriebsgerät eingesteckt werden. Dabei rasten Arretierstücke 30, 31 des distalen Gehäuseteils 3 in zugeordneten Halteschlitzen 32, 33 am Gegensteckteil 8 ein, so daß der Stecker 1 am Betriebsgerät arretiert ist. Die am nicht angesteckten Ende des Kombinationsschlauches 11, 11' angeordnete (nicht dargestellte) Saugelektrode ist jetzt sowohl pneumatisch als auch elektrisch über ein und denselben Stecker am Betriebsgerät angeschlossen.

## Patentansprüche

1.  Pneumatischer und elektrischer Steckanschluß zum Anschließen von Saugelektroden an ein Bedienungsgerät, insbesondere für Reizstrom-therapie, wobei ein elektrischer Mehrfachstek-ker (1) mit einem pneumatischen Rohranschluß (5) versehen ist, an dem ein Kombinationsschlauch (11 bzw. 11') angeschlossen ist, der aus einem mit dem pneumatischen Teil der Saugelektroden verbundenen pneumatischen Schlauchteil (12) und aus im Innern des pneumatischen Schlauchteiles oder am äußeren Schlauchmantel entlang geführten Leitungen (14 bzw. 20 bis 22), die mit dem elektrischen Teil der Saugelektroden verbunden sind, besteht und wobei ein Gegensteckteil (8) am Bedienungsgerät einen pneumatischen Gegenanschluß (7) für den pneumatischen Rohranschluß (5) sowie Gegensteckbuchsen (9) für an den Leitungen (14 bzw. 20 bis 22) des Mehrfachsteckers (1) vorgesehene Steckstifte (4) aufweist.

2.  Steckanschluß nach Anspruch 1, wobei das pneumatische Schlauchteil (12) von einem elektrischen Schlauchteil (18) überzogen ist und wobei wenigstens ein elektrisches Kabel (20 bis 22) zwischen dem inneren pneumatischen Schlauchteil und dem äußeren elektrischen Schlauchteil gelagert ist.

3.  Steckanschluß nach Anspruch 2, wobei für mehrpoligen Anschluß eine Mehrzahl von Kabel (20 bis 22) zwischen pneumatischem Schlauchteil (12) und elektrischem Schlauchteil (18) gelagert ist.

4.  Steckanschluß nach einem der Ansprüche 1 bis 3, wobei für einen einpoligen elektrischen Anschluß eine Metallader (15) einer einpoligen elektrischen Leitung (14) an einen metallischen pneumatischen Rohranschluß (5) über eine Andruckmuffe (16) elektrisch angeschlossen ist und der metallische Rohranschluß (5) durch einen elektrischen Anschluß (17) mit Kontaktteilen (Steckstiften 4) des elektrischen Stek-kers (1) verbunden ist.

## Claims

1.  Pneumatic and electrical plug-in connector for connecting suction electrodes to a control unit, in particular for stimulus current therapy, an electrical multiplug (1) being provided with a pneumatic pipe connector (5) to which a combination hose (11 or 11') is connected which comprises a pneumatic hose portion (12) connected to the pneumatic portion of the suction electrodes, and conductors (14 or 20 to 22), guided inside the pneumatic hose portion or along the outer hose casing, which are connected to the electrical portion of the suction electrodes, and wherein a mating plug-in portion (8) on the control unit has a pneumatic mating connection (7) for the pneumatic pipe connector (5), as well as mating plug-in sockets (9) for plug-in pins (4) provided on the conductors (14 or 20 to 22) of the multiplug (1).

2.  Plug-in connector according to claim 1, wherein the pneumatic hose portion (12) is surrounded by an electrical hose portion (18) and wherein at least one electrical cable (20 to 22) is mounted between the inner pneumatic hose portion and the outer electrical hose portion.

3. Plug-in connector according to claim 2, wherein a plurality of cables (20 to 22) is mounted between pneumatic hose portion (12) and electrical hose portion (18) for multi-pole connection.

4. Plug-in connector according to one of claims 1 to 3, wherein for a single-pole electrical connection a metal core (15) of a single-pole electrical conductor (14) is electrically connected to a metallic pneumatic pipe connector (5) by means of a pressure sleeve (16), and the metallic pipe connector (5) is connected by an electrical connection (17) to contact portions (plug-in pins 4) of the electrical plug connector (1).

**Revendications**

1. Raccord à enfichage pneumatique et électrique pour le raccordement d'électrodes de succion à un appareil de service, notamment pour la thérapie par courants de stimulation, dans lequel un connecteur électrique multiple (1) est pourvu d'un raccord tubulaire pneumatique (5), auquel est raccordé un tuyau combiné (11 ou 11') qui est constitué par un élément de tuyau pneumatique (12) raccordé à la partie pneumatique des électrodes de succion, et par des conducteurs (14 ou 20 à 22) qui s'étendent à l'intérieur de l'élément de tuyau pneumatique ou sur l'enveloppe extérieure du tuyau et sont raccordés à la partie électrique des électrodes de succion, et dans lequel un élément d'enfichage antagoniste (8) présent sur l'appareil de service possède un raccord pneumatique antagoniste (7) pour le raccord tubulaire pneumatique (5) ainsi que des douilles antagonistes (9) pour les fiches mâles (4) prévues sur les conducteurs (14 ou 20 à 22) du connecteur multiple (1).

2. Raccord à enfichage suivant la revendication 1, dans lequel l'élément de tuyau pneumatique (12) est recouvert par un élément de gaine électrique (18) et dans lequel au moins un câble électrique (20 à 22) est monté entre l'élément de tuyau pneumatique intérieur et l'élément de gaine électrique extérieur.

3. Raccord à enfichage suivant la revendication 2, dans lequel pour un raccordement multipolaire, une multiplicité de câbles (20 à 22) sont disposés entre l'élément de tuyau pneumatique (12) et l'élément de gaine électrique (18).

4. Raccord à enfichage suivant l'une des revendications 1 à 3, dans lequel, pour un raccordement électrique monopolaire, un fil métallique (15) d'un conducteur électrique unipolaire (14) est raccordé électriquement à un raccord tubulaire pneumatique métallique (5) par l'intermédiaire d'un manchon de serrage (16), et le raccord tubulaire métallique (5) est raccordé au moyen d'un raccord électrique (17) à des pièces de contact (fiches mâles 4) du connecteur électrique (1).

FIG 1

FIG 2

FIG 3

FIG 4

FIG 5